# EUROPÄISCHE PATENTSCHRIFT

(11) **EP 0 775 709 B1**
(45) Veröffentlichungstag und Bekanntmachung des Hinweises auf die Patenterteilung: **26.03.2003**
(21) Anmeldenummer: 96118002.3
(22) Anmeldetag: 09.11.1996
(51) Int. Cl.: C07H 3/04, C07H 7/033, C07C 51/235, B01J 23/42, C07B 41/08, B01D 61/44

(54) **Verfahren zur Herstellung von di- und höheroxidierten Carbonsäuren von Kohlenhydraten, Kohlenhydratderivaten oder primären Alkoholen**
Method for preparing di- and higher oxidised carboxylic acids of carbohydrates, carbohydrate derivatives or primary alcohols
Méthode pour préparer des acides carboxyliques dioxydés ou polyoxydés d'hydrates de carbones, des dérivés d'hydrates de carbone ou des alcools

(30) Priorität: 14.11.1995 DE 19542287
(43) Veröffentlichungstag der Anmeldung: 28.05.1997
(73) Patentinhaber: SÜDZUCKER AKTIENGESELLSCHAFT MANNHEIM/OCHSENFURT, 68165 Mannheim (DE)
(72) Erfinder: Kunz, Markwart, Dipl.-Chemiker, 67550 Worms (DE); Schwarz, Andreas, Dipl.-Chemiker, 94447 Plattling (DE); Kowalczyk, Jörg, Dr., 67248 Bockenheim (DE)
(74) Vertreter: Einsel, Martin, Dipl.-Phys.

(56) Entgegenhaltungen:
- EP-A- 0 040 709
- EP-A- 0 206 054
- EP-A- 0 218 150
- EP-A- 0 326 673
- DE-A- 3 926 642
- DE-A- 4 307 388

## Beschreibung

Die Erfindung betrifft ein Verfahren zur Herstellung von di- und höheroxidierten Carbonsäuren von Kohlenhydraten, Kohlenhydratderivaten oder primären Alkoholen. Sie betrifft ferner eine Vorrichtung zur Durchführung des Verfahrens.

Es existieren verschiedene Methoden, Kohlenhydrate selektiv an zwei Positionen zu oxidieren. Beispielsweise erhält man Glucarsäure als Aldarsäure durch Oxidation von Glucose mit konzentrierter Salpetersäure. Durch die rauhen Bedingungen kann man mit dieser absatzweise zu betreibenden Reaktion jedoch nur geringe Ausbeuten an Kaliumhydrogenglucarat erhalten [C. L. Mehltretter, D-Glucavic Acid, in: Methods Carbohydr. Chem. 2 (1963), 46 ].

Ebenfalls ist ein Aspergillus niger - Stamm beschrieben, der D-Glucose zu D-Glucarat oxidiert [F. Challenger et al., Formation of Organic Acids from Sugars by Aspergillus niger, in: Nature 119 (1927), 674]. Biotechnologische Verfahren weisen jedoch einige gravierende Nachteile auf. Die Anzucht der Mikroorganismen bzw. Herstellung der Biokatalysatoren ist problematisch, da man für Bioreaktoren meist Reinkulturen benötigt. Folglich ergibt sich oft die Notwendigkeit einer sterilen Fahrweise des Prozesses, was erhebliche Anlagekosten bedeutet. Aufgrund der genetischen Instabilität von Mikroorganismen ist es häufig technisch unmöglich, ein kontinuierliches Produktionsverfahren zu realisieren.

Eine weitere Möglichkeit, zu dioxidierten Kohlenhydraten zu gelangen, stellt die heterogen katalysierte Oxidation mit Edelmetallen der 8. Nebengruppe auf entsprechenden Trägermaterialien dar. Die Oxidation von D-Glucose oder von D-Gluconsäure zur D-Glucarsäure mit Luftsauerstoff erfolgt dabei chemisch z.B. an Pt/C-Katalysatoren. Nachteilig bei dieser absatzweisen Reaktionsführung ist dabei die geringe Selektivität bezüglich Glucarsäure. Das entstehende Produktgemisch ist vielmehr sehr komplex. Unter optimierten Bedingungen beträgt die Ausbeute an Kalium-glucarat lediglich ca. 40 % nach Kristallisation [H. Röper, Selective Oxidation of D-Glucose: Chiral Intermediates for Industrial Utilization, in: Starch/ Stärke 42 (1990), Nr. 9, 342-349].

Bei der Oxidation von Saccharose treten ähnliche Probleme auf. Bereits HEYNS und PAULSEN untersuchten diese Reaktion an Platinkatalysatoren [K. Heyns and H. Paulsen, Selective Catalytic Oxidation of Carbohydrates, Employing Platinum Catalysts, in: Adv. Carbohydr. Chem. 17 (1962), 169]. Die Produktzusammensetzungen waren hinsichtlich chemischer Strukturen und Zusammensetzungen so komplex, daß keine näheren Angaben gemacht wurden.

Neuere Untersuchungen [L. A. Edye et al., Platinum Catalysed Oxidation of Sucrose, in: J. Carbohydr. Chem. 10 (1) (1991), 11; C. Recker, Dissertation, TU Braunschweig (1995)] haben ergeben, daß das Produktgemisch neben den drei denkbaren Monooxidationsprodukten auch C₆₆-Saccharosedicarbonsäure und andere, nicht identifizierbare Substanzen enthält.

Ein in der DE 35 35 720 A1 und in der EP 0 218 150 A2 beschriebenes Verfahren zur katalytischen Oxidation von Saccharose weist darauf hin, daß im Produktgemisch neben anderen höheroxidierten Produkten auch Saccharosetricarbonsäure zu finden ist. Ein Verfahren zur Herstellung dieser Saccharosetricarbonsäure wird dabei vorgeschlagen, bei welchem Saccharose mit Sauerstoff ggf. im Gemisch mit inerten Gasen mit Hilfe eines effektiven Katalysators oxidiert wird. Es muß sich dabei um einen effektiveren Katalysator als Platin/Tonerde handeln. Erwähnt sind dabei Palladium, aber auch Platin selbst, auf Aktivkohlen, vor allem den aktiveren Typen. Bevorzugt wird hochkonzentrierter Sauerstoff zur Oxidierung verwendet. Das Verfahren ist nicht kontinuierlich und wirtschaftlich nicht durchführbar.

Zur selektiven Herstellung von Monocarbonsäuren von Kohlenhydraten, Kohlenhydratderivaten oder primären Alkoholen ist es aus der DE 43 07 388 A1 bekannt, die Ausgangsstoffe einem Oxidationsreaktor zuzuführen und eine Elektrodialyseeinheit nachzuschalten, in der die Monooxidationsprodukte gewonnen werden. Die nichtoxidierten Stoffe werden kontinuierlich in den Oxidationsreaktor zurückgeführt. Die Selektivität der Reaktion bezüglich Monocarbonsäurebildung ist ausgezeichnet, di- bzw. höheroxidierte Carbonsäuren sind mit diesem Verfahren jedoch nicht herzustellen.

Aufgabe der Erfindung ist es dem gegenüber, eine Oxidation von Kohlenhydraten, Kohlenhydratderivaten und primären Alkoholen mit einer besseren Selektivität bezüglich der di- und höheroxidierten Produkte vorzuschlagen.

Diese Aufgabe wird dadurch gelöst, daß man kontinuierlich Kohlenhydrate, Kohlenhydratderivate oder primäre Alkohole oder monooxidierte Kohlenhydrate, Kohlenhydratderivate oder primäre Alkohole in wäßriger Lösung und Konzentrationen zwischen 0,1 und 60 % und mit Sauerstoff oder sauerstoffhaltigen Gasen an Edelmetall- oder Mischmetallkatalysatoren oxidiert, den Volumenstrom der so gebildeten Produkte einer oder mehreren in Reihe geschalteten Elektrodialysestufen zuführt und dort die di- und höheroxidierten Carbonsäuren entfernt und gewinnt.

Dieses Verfahren ist hervorragend geeignet, die Aufgabe zu lösen. Dabei wird von einem überraschenden Effekt Gebrauch gemacht: Das aus der DE 43 07 388 A1 bekannte Verfahren ist wie schon erwähnt dazu vorgesehen, Monocarbonsäuren zu gewinnen. Es schafft dies auch mit einer sehr hohen Selektivität. Die Fachkreise gingen bisher auch in jüngsten Publikationen noch davon aus, daß Dicarbonsäuren dabei nicht entstehen oder gar gewonnen werden könnten [M. Kunz et al., Katalytische Oxidation von Isomaltulose, in: Chem. Ing. Tech. 67 (1995), Nr. 7, 836].

Werden die bei diesem Verfahren entstehenden Monocarbonsäuren jedoch nicht selektiv abgetrennt, sondern statt dessen weitgehend in den Oxidationsprozeß zurückgeführt, so entstehen bei diesem Prozeß entgegen dieser bisherigen Auffassung doch Dicarbonsäureanteile, dieser Anteil entsteht vermehrt bei ständig wiederholter Zurückführung.

Genau diese Dicarbonsäureanteile können mit einer Elektrodialysestufe jedoch ebenfalls selektiv abgetrennt werden. Der Einsatz von Elektrodialyse bei der Gewinnung von Säuren aus Salzen ist zwar aus beispielsweise der DE 3 926 642 A1 bekannt, nicht jedoch der nunmehrige Anwendungsbereich. Zur Abtrennung der eben erwähnten Dicarbonsäureeanteile macht man sich die überraschende Tatsache zu Nutzen, daß die Elektrodialyse als Membrantrennverfahren in der Lage ist, zwischen Mono- und Dianionen bzw. zwischen Ionen mit unterschiedlichen Ladungseigenschaften zu diskriminieren.

Dadurch wird dieses Verfahren zur Darstellung und Gewinnung von Dicarbonsäuren hervorragend geeignet. Die gebildeten Disäuren können kontinuierlich aus dem Oxidationskreislauf entfernt werden und gehen keine unerwünschten Folgereaktionen mehr ein, die die Selektivität der Reaktion bezüglich der Disäurebildung herabsetzen würden. Durch Rückführung des Gemisches von Monooxidationsprodukten und dem als Edukt eingesetzten Kohlenhydrat zur Oxidationsstufe ergibt sich die kontinuierliche Prozeßführung. Entsprechend dem Abzug an Dicarbonsäuren aus dem Reaktionskreislauf regelt man die Eduktzugabe so ein, daß die Eduktkonzentration konstant bleibt. Bei dieser Reaktionsführung treten die Monocarbonsäuren als Zwischenprodukte auf, für die sich eine stationäre Konzentration im Reaktionskreislauf einstellt.

Insofern hat man mit der Erfindung ein kontinuierliches Verfahren zur selektiven Produktion von Dicarbonsäuren vorliegen, das bezüglich Selektivität und Reaktionsführung den bisher bekannten Verfahren zur Herstellung von Dicarbonsäuren deutlich überlegen ist.

Solche spezifisch di- oder höherfunktionalisierten Saccharide bzw. Saccharidderivate sind von hohem industriellen Interesse, da sie einerseits wegen ihrer Hydrophilie, ihrer Hautverträglichkeit und ihren komplexbildenden Eigenschaften direkte Applikationsmöglichkeiten besitzen, andererseits durch einfache, technisch durchführbare Derivatisierungen zu interessanten Produkten auf dem Polymer- und Tensidsektor weiterverarbeitet werden können. Durch ihre ökologisch positiven Eigenschaften weisen diese Produkte gegenüber bekannten Wettbewerbsprodukten, die auf der Petrochemie basieren, erhebliche Vorteile auf.

Das Ergebnis des Verfahrens war recht überraschend, da das technische Verfahren aus der DE 43 07 388 A1 ja gerade im Gegenteil dazu gedacht ist, die Dicarbonsäureanteile in einem Oxidationsgemisch aus Kohlenhydraten, Kohlenhydratderivaten oder primären Alkoholen möglichst zugunsten einer selektiven Monocarbonsäureproduktion einzuschränken und dieses Ziel ja auch im höchsten Maße erreicht wird. Der Erfolg wird dadurch möglich, daß durch die Rückführung der Monosäureprodukte sich ein sehr hoher Zwischenproduktkonzentrationspegel von monooxidierten Produkten einstellt, die dadurch dann doch letztlich zu einer Dicarbonsäureproduktion führen.

Zur Gewinnung der Dicarbonsäure ist in der Elektrodialysestufe ein Spannungswert bevorzugt, der deutlich niedriger als die sonst in Elektrodialysestufen eingesetzten Spannungen liegt und auch gerade deutlich niedriger als derjenige Wert ist, der zur Abtrennung der Monocarbonsäuren im Stand der Technik nach der DE 43 07 388 A1 verwendet wird. Experimente haben ergeben und bestätigt, daß durch diese sehr niedrige Spannung eine Selektion gerade zwischen den Monocarbonsäuren und den Dicarbonsäuren stattfinden kann, also die Dicarbonsäuren herausgefiltert werden. In den Experimenten ist dabei ein Spannungswert von 0,1 bis 4,9 Volt als besonders geeignet aufgetreten. Es kann aber bei der Verwendung anderer Membrantypen auch ein höherer Wert bevorzugt werden, da es gerade auf die relativen Verhältnisse zu den bei der Abtrennung von Monocarbonsäuren verwendeten Spannungen ankommt, die bei anderen Membrantypen auch höher liegen.

Besonders bevorzugt ist es, wenn mehrere Elektrodialysestufen hintereinander geschaltet werden. Durch dieses Hintereinanderschalten wird eine erhebliche Verbesserung der Selektivität erzielt.

Eine weitere Verbesserung und vor allem eine Steigerung der Geschwindigkeit der Dicarbonsäurebildung erfolgt bevorzugt dadurch, daß für die Katalysatoren poröse Trägermaterialien bzw. solche mit großer spezifischer Oberfläche eingesetzt werden.

Die Steigerung der Geschwindigkeit der Dicarbonsäurebildung durch derartige Katalysatoren wurde ebenfalls experimentell bestätigt. Auch durch die bei der Darstellung von Monocarbonsäuren bevorzugten Katalysatoren läßt sich die gewünschte Dicarbonsäure herstellen, die höhere Bildungsgeschwindigkeit ist jedoch auch für die schnellere bzw. selektivere Gewinnung der Dicarbonsäure nach der Elektrodialysestufe von Vorteil.

Im folgenden soll an Hand der Figuren ein Ausführungsbeispiel im einzelnen beschrieben werden. Es zeigen:
- **Fig. 1**: eine bevorzugte Ausführungsform der Erfindung und
- **Fig. 2**: einen Verfahrensablauf zum Ausführungsbeispiel.

Bei dem Ausführungsbeispiel wird konkret die katalytische Oxidation der Saccharose zu den Saccharosedicarbonsäuren diskutiert.

Das Saccharosemolekül besitzt drei primäre Hydroxygruppen, die allesamt zur Carboxylfunktion oxidiert werden können. Die daraus abzuleitenden Carbonsäuren werden im folgenden mit ihren Kurzbezeichnungen geführt, welche bedeuten:

| | |
|---|---|
| C₆-Saccharosemonocarbonsäure | 2-O-(1-O-α-D-Glucopyranosyl)-β-D-fructofuranuronsäure |
| | |
| C_{6'}-Saccharosemonocarbonsäure | 1-O-(2-O-β-D-Fructofurannosyl)-α-D-glucopyranuronid |
| | |
| C₁-Saccharosemonocarbonsäure | 2-O-(1-O-α-D-Glucopyranosyl)-β-D-arabino-2-hexulofuranonsäure |
| | |
| C_{66'}-Saccharosedicarbonsäure | 1-O-(2-O-β-D-Fructofuranuronyl)-α-D-glucopyranuronid |
| | |
| C₆₁-Saccharosedicarbonsäure | 2-O-(1-O-α-D-Glucopyranosyl)-β-D-arabino-2-hexulofuranarsäure |
| | |
| C_{6'1}-Saccharosedicarbonsäure | 1-O-(2-O-β-D-arabino-2-Hexulofuranosonyl)-α-D-glucopyranuronid |
| | |
| C_{66'1}-Saccharosetricarbonsäure | 2-O-(1-O-α-D-Glucopyranuronyl)-β-D-arabino-2-hexulofuranarsäure |

In ein Rührgefäß 10 mit einem Rührer 11 und einem Motor 12 für den Rührer 11 wird eine wäßrige Saccharoselösung gegeben, die durch den Frittenboden mit Luft zugeführt bei 21 begast wird. Mittels einer pH-Regelung 17 wird der pH-Wert im Rührgefäß 10 auf dem Sollwert gehalten. Nach Durchmischung im Rührgefäß wird die mit Sauerstoff angereicherte, thermostatisierte, pH-geregelte wäßrige Saccharoselösung über eine mit P bezeichnete Pumpe einer Oxidationsstufe 30 zugeführt, die in diesem Beispiel einen Pt/C-Trägerkatalysator enthält. In dieser Oxidationsstufe 30 findet die katalytische Oxidation der Saccharose zu den Monocarbonsäuren bzw. die Oxidation der Monocarbonsäuren zu den Dicarbonsäuren statt. Das Reaktionsgemisch wird anschließend über die nächste Pumpe zu einer Elektrodialysestufe 40 geführt. Die Elektrodialysespannung ist jedoch so niedrig gewählt, daß die Monocarbonsäuren nicht bzw. deutlich langsamer als die Saccharosedicarbonsäuren abgeführt und im Konzentratkreislauf über einen mit 41 bezeichneten Weg angereichert werden. Durch die Erniedrigung der Elektrodialysespannung findet also eine deutliche Diskriminierung zwischen Mono- und Dicarbonsäuren statt. Wird die so mit Dicarbonsäuren angereicherte Lösung erneut elektrodialysiert, findet eine weitere Anreicherung der Dicarbonsäuren statt. Je nach Anreicherung kann man so viele Elektrodialysestufen in Reihe schalten, daß die Dicarbonsäuren vollständig von den Monocarbonsäuren getrennt werden.

Die Monocarbonsäuren werden anschließend zusammen mit nichtoxidierter Saccharose über den Weg 43 wieder in den Behälter bzw. das Rührgefäß 10 zurückgepumpt, von wo aus sie nach erneuter Durchmischung, Luftanreicherung und pH-Regelung wieder in die Oxidationsstufe 30 gepumpt werden.

Die kontinuierliche Arbeitsweise der Apparatur wird dadurch erreicht, daß entsprechend dem Saccharoseverbrauch das Edukt nachdosiert wird, so daß dessen Konzentration nahezu konstant ist.

Bei dem hier verwendeten Katalysator wurde die Saccharose schneller zu den Monocarbonsäuren oxidiert als die Monocarbonsäuren zu den Disäuren, folglich reicherten sich die Monocarbonsäuren bis zu einer bestimmten stationären Konzentration im Reaktionskreislauf an. Diese Konzentration ist abhängig von der zur Oxidation benutzten Katalysatorenmenge, von der in der Elektrodialyseeinheit zur Verfügung stehenden Membranfläche und von der angelegten Spannung.

In einem Behälter 50 reicherte sich ein Produktgemisch an, das zu 56% aus Saccharosemonocarbonsäuren und zu 44% aus höheroxidierten Derivaten bestand. Hauptbestandteil dieser höheroxidierten Derivate war C_{66'}-Saccharosedicarbonsäure. Die beiden anderen Saccharosedicarbonsäuren (C₆₁- bzw. C_{6'1}-Saccharosedicarbonsäure) lagen jeweils zu 7,5%-Anteil am Produktgemisch vor. Nebenprodukte konnten nicht beobachtet werden. Das nach der zweiten Elektrodialysestufe erhaltene Gemisch bestand bereits zu 75% aus Saccharosedicarbonsäuren. Die Diskriminierung zwischen Dicarbonsäuren und Monocarbonsäuren in einer Elektrodialysestufe ist in hohem Maße von der angelegten Spannung abhängig. In einem typischen Versuch reicherten sich die Saccharosedicarbonsäuren ca. drei- bis viermal so schnell wie die Saccharosemonocarbonsäuren an.

Neben der auf diese Weise erzielbaren hohen Selektivität der Oxidation bezüglich der Bildung von Dicarbonsäuren - ohne daß diese Selektivität in den einzelnen Elektrodialysestufen in merklicher Weise durch Neben-, Folge- oder Abbauproduktbildung verringert wird - fällt als weiterer Verfahrensvorteil auf, daß der Katalysator auch in einem dreißigtägigen Langzeitversuch nicht desaktiviert.

Alternativ zur Saccharose als Edukt zur Dicarbonsäureproduktion kann ein Gemisch der Saccharosemonocarbonsäuren dienen, welches man mit dem zur Monocarbonsäureproduktion beschriebenen Verfahren problemlos herstellen und ohne weitere Aufreinigung zur Dicarbonsäureproduktion einsetzen kann. Die kontinuierliche Verfahrensweise ergibt sich in diesem Fall durch Nachdosierung des Monocarbonsäuregemisches dem Verbrauch entsprechend. Im Falle der Saccharosedicarbonsäureproduktion erweist es sich hinsichtlich der Reaktionsgeschwindigkeit als unerheblich, ob als Edukt Saccharose oder ein Gemisch von Saccharosemonocarbonsäuren eingesetzt wird. Die Untersuchung der absatzweise ohne Elektrodialyse betriebenen katalytischen Oxidation von Palatinose ergab jedoch, daß die Sekundäroxidation der Monocarbonsäuren zu der Palatinosedicarbonsäure durch das Edukt der Primärreaktion gehemmt wird ( H. Puke, Dissertation, TU Braunschweig, 1992). In diesem Fall bietet es sich an, die Palatinosemonocarbonsäuren als Edukte für die kontinuierliche Palatinosedicarbonsäureproduktion einzusetzen, was zu einer Steigerung der Oxidationsgeschwindigkeit führt.

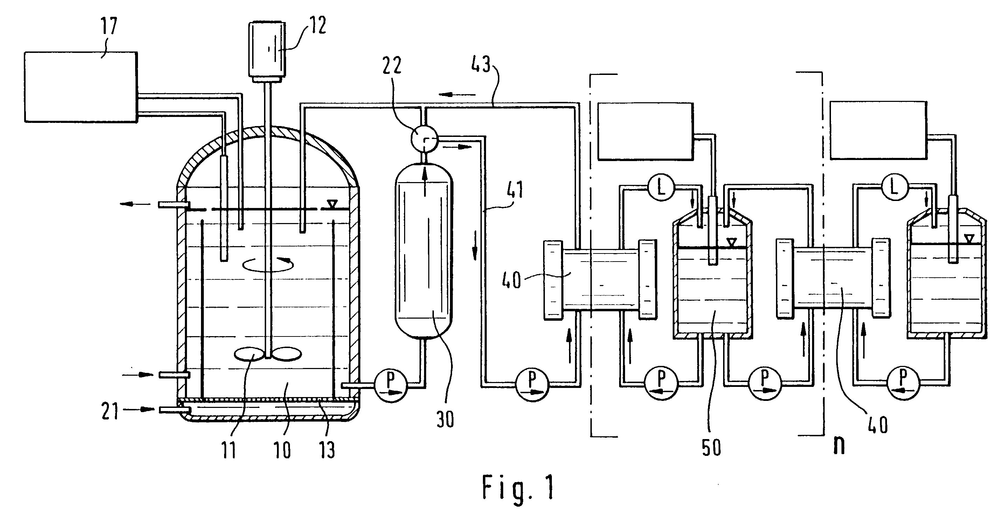

Die Möglichkeit einer solchen Verfahrensweise - katalytische Oxidation ohne Elektrodialyseeinheit - ist dabei in Figur 1 mit angedeutet; durch Umlegen eines Dreiwegehahns 22 wird die Reaktionslösung direkt nach Verlassen des Oxidationsreaktors 30 in das Rührgefäß 10 zurückgepumpt.

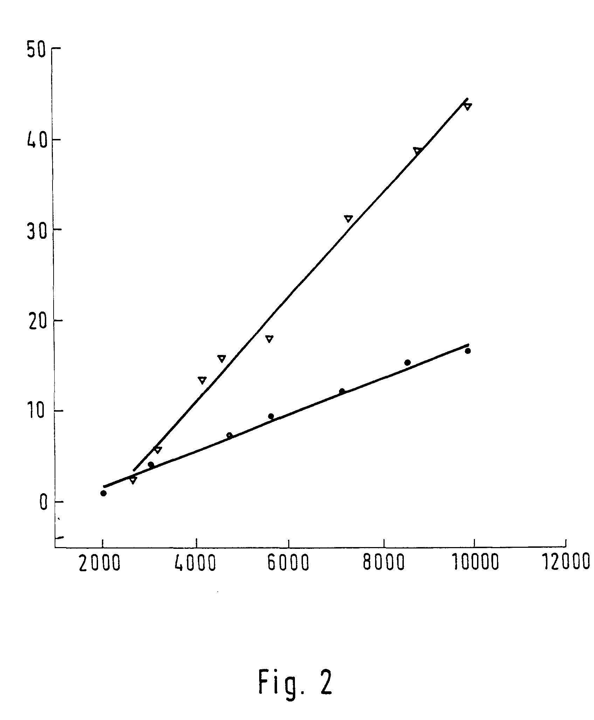

Bei der Saccharoseoxidation hat es sich gezeigt, daß beispielsweise durch Wahl einer Aktivkohle mit einer hohen spezifischen Oberfläche gegenüber einer Aktivkohle mit geringerer spezifischer Oberfläche, sich die Geschwindigkeit der kontinuierlichen Disäureproduktion beträchtlich steigern läßt. In Figur 2 wird die zeitliche Zunahme der C_{66'}-Saccharosedicarbonsäure in Abhängigkeit von zwei verwendeten Katalysatoren A und B dargestellt. Nach rechts ist die Reaktionszeit in Minuten, nach oben die C_{66'}-Saccharosedicarbonsäure in mmol aufgetragen. Die beiden Katalysatoren werden durch unterschiedliche Symbole angegeben, nämlich der Katalysator A durch schwarze Punkte •, der Katalysator B durch leere Dreiecke ∇. Bei den beiden Katalysatoren handelt es sich um Pt/C-Trägerkatalysatoren, die eine ähnliche Korngrößenverteilung aufweisen. Die als Trägermaterialien verwendeten Aktivkohlen unterscheiden sich jedoch hinsichtlich ihrer spezifischen Oberfläche, wobei Katalysator B die größere spezifische Oberfläche aufweist.

Das am Beispiel der Saccharoseoxidation beschriebene Verfahren der kontinuierlichen Dicarbonsäureproduktion läßt sich ohne Schwierigkeiten auf andere Kohlenhydrate und -derivate übertragen.

Bei der Oxidation von D-Glucose oder D-Gluconsäure läßt sich die Glucarsäure ebenfalls durch Erniedrigung der Elektrodialysespannung bevorzugt vor den beiden Monooxidationsprodukten Gluconsäure und Glucuronsäure im Konzentratkreislauf anreichern. Die Selektivität der Reaktion bezüglich Glucarsäure liegt dabei deutlich höher als bei der absatzweisen Reaktionsführung, da die Glucarsäure aus dem Oxidationskreislauf entfernt wird, bevor Folgereaktionen auftreten können.

Die aufgeführten Beispiele belegen, daß das Verfahren der kontinuierlichen katalytischen Oxidation von Kohlenhydraten, - derivaten und primären Alkoholen unter Abtrennung der Reaktionsprodukte mit Hilfe einer Elektrodialyse nicht nur für die Monocarbonsäureproduktion geeignet ist, sondern nach Variation einiger Betriebsparameter ebenso auf die selektive Dicarbonsäureproduktion angewendet werden kann.

Weiterhin ist es möglich, durch weitere Absenkung der Elektrodialysespannung zwischen Di- und Tricarbonsäuren zu diskriminieren. In einem Experiment wurden die durch mehrstufige Elektrodialyse aufgereinigten Saccharosedicarbonsäuren bei einer Elektrodialysespannung von 1,0 V oxidiert, wodurch es zu einer Anreicherung von Saccharosetricarbonsäure im Konzentratkreislauf kam. Durch mehrstufige Elektrodialyse kann das Edukt abgetrennt werden. Die Oxidationsgeschwindigkeit war bei dem verwendeten Katalysator jedoch sehr langsam, ferner wurde die Bildung von Spalt- und Abbauprodukten der Saccharosedicarbonsäuren beobachtet.

### Beispiel 1:

### Kontinuierliche Oxidation von Saccharose bei 35°C.

In der beschriebenen Apparatur werden im Oxidationskreislauf 40 g Platin/ Aktivkohlekatalysator (1% Pt/C; Korngröße 40 - 100 µm, Fa. Degussa) eingesetzt, durch die 1500 ml einer 0,1 molaren wäßrigen Saccharoselösung gepumpt werden. Der Oxidationskreislauf entspricht dem Diluatkreislauf der Elektrodialyse. Für den Konzentratkreislauf wird destilliertes Wasser und als Elektrodenspüllösung 1 M Na₂SO₄ eingesetzt. Die Reaktionstemperatur wird mit Hilfe eines Umlaufthermostaten auf 35°C gehalten. Die Begasung des Rührkessels wird mit Hilfe von Rotametern auf 100 cm³ O₂/min und 400 cm³ N₂/min eingestellt. Der pH-Sollwert im Dialysatkreislauf wird durch Titration der entstehenden Säuren mittels 1 M K₂CO₃ auf pH 6,5 gehalten. Die Elektrodialyse (Bel 11, Fa. Berghof GmbH Labortechnik) ist mit 6 AMV/CMV Membranpaaren (effektive Membranfläche = 360 cm²) ausgestattet und wird bei einer Spannung von 2,0 Volt betrieben. Der Reaktionsverlauf wird durch HPLC-Messungen verfolgt. Das Produktspektrum, das sich im Konzentrat anreichert, hat folgende Zusammensetzung:

| | |
|---|---|
| Saccharosemonocarbonsäuren | 56% |
| Saccharosedicarbonsäuren | 44% |

Das Produktgemisch wird gefriergetrocknet; 60 g davon werden in 500 ml destilliertem Wasser gelöst und erneut bei 2,0 Volt elektrodialysiert. Das Produktspektrum, das sich im Konzentrat anreichert, hat folgende Zusammensetzung:

| | |
|---|---|
| Saccharosemonocarbonsäuren | 25 % |
| Saccharosedicarbonsäuren | 75% |

Die Elektrodialyse wurde abgebrochen bei einer Diluatzusammensetzung von:

| | |
|---|---|
| Saccharosemonocarbonsäuren | 92% |
| Saccharosedicarbonsäuren | 8% |

### Beispiel 2:

Oxidation der Saccharose in Analogie zu Beispiel 1 bei einer Elektrodialysespannung von 2,5 Volt. Das erhaltene Produktgemisch hat folgende Zusammensetzung:

| | |
|---|---|
| Saccharosemonocarbonsäuren | 34% |
| Saccharosedicarbonsäuren | 66% |

### Beispiel 3:

Oxidation eines Gemisches der drei Saccharosemonocarbonsäuren
- (Zusammensetzung:: C₆-Saccharosemonocarbonsäure: 44%
C_{6'}-Saccharosemonocarbonsäure: 36%
C₁-Saccharosemonocarbonsäuren: 20%)
in Analogie zu Beispiel 1 bei einer Elektrodialysespannung von 2,0 Volt. Das erhaltene Produktgemisch hat dieselbe Zusammensetzung wie bei Beispiel 1:

| | |
|---|---|
| Saccharosemonocarbonsäuren | 56% |
| Saccharosemicarbonsäuren | 44% |

### Beispiel 4:

Kontinuierliche Oxidation von Na-D-gluconat.

Die Oxidation des Na-D-gluconats erfolgt in der in Beispiel 1 beschriebenen Apparatur, wobei die AMV-Membranen des Elektrodialysestacks durch AM3-Membranen ersetzt sind und die Elektrodialysespannung bei 1,3 V liegt. Die Na-D-gluconatkonzentration beträgt 0,1 mol/l, pH-Wert und Temperatur sind auf 8,5 bzw. 35°C eingestellt. Die kontinuierlich erhaltene Produktlösung hat folgende Zusammensetzung:

| | |
|---|---|
| Gluconsäure | 60% |
| andere Monocarbonsäuren | 13% |
| Glucarsäure | 27% |

Demnach beträgt die Selektivität der Reaktion bezüglich Glucarsäure 68%. In der Eduktlösung konnte NMR-spektroskopisch keine Glucarsäure nachgewiesen werden.

## Patentansprüche

1. Verfahren zur Herstellung von di- und höheroxidierten Carbonsäuren von Kohlenhydraten, Kohlenhydratderivaten oder primären Alkoholen, bei dem man kontinuierlich Kohlenhydrate, Kohlenhydratderivate oder primäre Alkohole oder monooxidierte Kohlenhydrate, Kohlenhydratderivate oder primäre Alkohole in wässriger Lösung und Konzentrationen zwischen 0,1 % und 60 % und mit Sauerstoff oder sauerstoffhaltigen Gasen an Edelmetall- oder Mischmetallkatalysatoren oxidiert, den Volumenstrom der so gebildeten Produkte einer oder mehreren in Reihe geschalteten Elektrodialysestufen zuführt und dort die di- und höheroxidierten Carbonsäuren entfernt und gewinnt.

2. Verfahren nach Anspruch 1,
**dadurch gekennzeichnet,**
**daß** man nicht- oder monooxidierte Edukte nach dem Entfernen der di- und höheroxidierten Carbonsäuren der Oxidationsstufe wieder zuführt.

3. Verfahren nach Anspruch 1 oder 2,
**dadurch gekennzeichnet,**
**daß** die Elektrodialyse mit einer Spannung in einem Bereich durchgeführt wird, der niedriger als der zur Abtrennung von Monocarbonsäuren bei Elektrodialysestufen gleichen Typs liegt.

4. Verfahren nach einem der vorstehenden Ansprüche,
**dadurch gekennzeichnet,**
**daß** die Elektrodialyse mit einer Spannung im Bereich zwischen 0,1 Volt und 7 Volt.

5. Verfahren nach Anspruch 4,
**dadurch gekennzeichnet,**
**daß** die Elektrodialyse mit einer Spannung im Bereich zwischen 0,1 Volt und 4,9 Volt durchgeführt wird.

6. Verfahren nach einem der vorstehenden Ansprüche,
**dadurch gekennzeichnet,**
**daß** als Membranen in der Elektrodialyse lonenaustauscher- oder bipolare Membranen zum Einsatz kommen.

7. Verfahren nach einem der vorstehenden Ansprüche,
**dadurch gekennzeichnet,**
**daß** die Elektrodialyse in mehreren in Reihe geschalteten Elektrodialysestufen durchgeführt wird, bei denen jeweils die di- und höheroxidierten Carbonsäuren angereichert und der nächsten Elektrodialysestufe zugeführt werden.

8. Verfahren nach einem der vorstehenden Ansprüche,
**dadurch gekennzeichnet,**
**daß** für die Katalysatoren poröse Trägermaterialien bzw. solche mit großer spezifischer Oberfläche eingesetzt werden.

9. Verfahren nach einem der vorstehenden Anspüche,
**dadurch gekennzeichnet,**
**daß** als Ausgangssubstanzen nichtreduzierende Saccharide, wie z.B. Saccharose, Trehalose und/oder reduzierende Saccharide, wie z.B. Glucose, Palatinose, Fructose, und/oder Zuckeralkohole, wie z.B. Palatinit, Sorbit, und/oder die monooxidierten Derivate bzw. Gemische oder monooxidierten Derivate der genannten Verbindungen, eingesetzt und dioxidiert werden.

10. Verfahren nach einem der vorstehenden Ansprüche,
**dadurch gekennzeichnet,**
**daß** als Lösungsmittel für die Ausgangssubstanzen Wasser oder Mischungen aus Wasser und sekundären Alkoholen, vorzugsweise Isopropanol, eingesetzt werden.

11. Verfahren nach einem der vorstehenden Ansprüche,
**dadurch gekennzeichnet,**
**daß** die Oxidation und die Elektrodialyse im pH-Bereich von 1 bis 13 erfolgen.

12. Verfahren nach einem der vorstehenden Ansprüche,
**dadurch gekennzeichnet,**
**daß** die Temperatur für die Oxidation zwischen 0°C und 80°C.

13. Verfahren nach Anspruch 12,
**dadurch gekennzeichnet,**
**daß** die Temperatur für die Oxidation zwischen 20°C und 60°C liegt.

14. Verfahren nach einem der vorstehenden Ansprüche,
**dadurch gekennzeichnet,**
**daß** man die Edukte in Konzentrationen zwischen 3% und 20% einsetzt.

15. Verfahren nach einem der vorstehenden Ansprüche,
**dadurch gekennzeichnet,**
**daß** zur pH-Einstellung Na₂CO₃, K₂CO₃ oder NaHCO₃ oder NaOH oder andere Alkalisierungsmittel eingesetzt werden.

16. Vorrichtung zur Durchführung eines der Verfahren nach einem der Ansprüche 1 bis 15,
**dadurch gekennzeichnet,**
**daß** sie in Reihe geschaltet eine Begasungsstufe (10), eine Oxidationsstufe (30) und mehrere Elektrodialysestufen (40) aufweist, sowie ferner eine Rückleitung (43) von den Elektrodialysestufen zur Begasungsstufe (10).

## Claims

1. A method for producing di- and more highly oxidized carboxylic acids from carbohydrates, carbohydrate derivatives or primary alcohols, in which carbohydrates, carbohydrate derivatives or primary alcohols or monooxidized carbohydrates, carbohydrate derivatives or primary alcohols are oxidized continuously in aqueous solution and concentrations between 0.1% and 60% and with oxygen or oxygen-containing gases on noble metal or mixed metal catalysts, the flow of the products so formed is passed to one or more electrodialysis stages connected in series and the di- and more highly oxidized carboxylic acids are removed and recovered there.

2. A method according to claim 1, **characterised in that** after the removal of the di- and more highly oxidized carboxylic acids, non- or monooxidized educts are passed to the oxidation stage again.

3. A method according to claim 1 or 2, **characterised in that** the electrodialysis is carried out with a voltage, which is in a range lower than that for the separation of monocarboxylic acids with electrodialysis stages of the same type.

4. A method according to any of the preceding claims, **characterised in that** the electrodialysis is carried out with a voltage in the range between 0.1 volts and 7 volts.

5. A method according to claim 4, **characterised in that** the electrodialysis is carried out with a voltage in the range between 0.1 volts and 4.9 volts.

6. A method according any of the preceding claims, **characterised in that** ion-exchange or bipolar membranes are used as membranes in the electrodialysis.

7. A method according to any of the preceding claims, **characterised in that** the electrodialysis is conducted in several electrodialysis stages connected in series, and in each stage the di- and more highly oxidized carboxylic acids are enriched and passed to the next electrodialysis stage.

8. A method according to any of the preceding claims, **characterised in that** porous support materials or ones with large specific surface are used for the catalysts.

9. A method according to any of the preceding claims, **characterised in that** non-reducing saccharides, such as saccharose, trehalose, and/or reducing saccharides, such as glucose, palatinose, fructose, and/or sugar alcohols, such as palatinol, sorbitol, and/or the monooxidized derivatives or mixtures or monooxidized derivatives of the above-mentioned compounds are used as starting substances and dioxidised.

10. A method according to any of the preceding claims, **characterised in that** there are used as solvents for the starting substances water or mixtures of water and secondary alcohols, preferably isopropanol.

11. A method according to any of the preceding claims, **characterised in that** the oxidation and the electrodialysis take place in the pH range from 1 to 13.

12. A method according to any of the preceding claims, **characterised in that** the temperature for the oxidation lies between 0 °C and 80 °C.

13. A method according to claim 12, **characterised in that** the temperature for the oxidation lies between 20 °C and 60°C.

14. A method according to any of the preceding claims, **characterised in that** educts are used in concentrations between 3% and 20%

15. A method according to any of the preceding claims, **characterised in that** Na₂CO₃, K₂CO₃ or NaHCO₃ or NaOH or other alkalizing agents are used for setting the pH.

16. An apparatus for carrying out the method according to any of claims 1 to 15, **characterised in that** it comprises, connected in series, a gassing stage (10), an oxidation stage (30) and a plurality of electrodialysis stages (40), as well as a return line (43) from said electrodialysis stages to said gassing stage (10).

## Revendications

1. Procédé de préparation d'acides carboxyliques di- et polyoxydés d'hydrates de carbone, de dérivés d'hydrates de carbone ou d'alcools primaires, dans lequel on oxyde en continu, les hydrates de carbone, dérivés d'hydrate de carbone ou alcools primaires ou des hydrates de carbone, dérivés d'hydrate de carbone ou alcools primaires monooxydés, en solution aqueuse et à des concentrations allant de 0,1 à 60% et avec de l'oxygène ou des gaz oxygénés sur des catalyseurs de métal noble ou de mischmétal, le courant en volume du produit ainsi formé est conduit par une ou plusieurs étapes d'électrodialyses disposées en série et on y sépare et obtient les acides carboxyliques di- ou polyoxydés.

2. Procédé suivant la revendication 1, **caractérisé en ce que** l'on ramène à nouveau l'étape d'oxydation, des réactifs monooxydés ou non oxydés après élimination des acides carboxyliques di- ou polyoxydés.

3. Procédé suivant la revendication 1 ou 2, **caractérisé en ce que** l'électrodialyse est réalisée avec une tension dans un intervalle qui se situe en dessous de celui pour la séparation des acides monocarboxyliques lors d'étapes d'électrodialyse de même type.

4. Procédé suivant l'une quelconque des revendications précédentes, **caractérisé en ce que** l'électrodialyse est réalisée à une tension dans l'intervalle allant de 0,1 Volt à 7 Volt.

5. Procédé suivant la revendication 4, **caractérisé en ce que** l'électrodialyse est réalisée à une tension dans l'intervalle allant de 0,1 Volt à 4,9 Volt.

6. Procédé suivant l'une quelconque des revendications précédentes, **caractérisé en ce que** l'on considère comme membrane dans l'électrodialyse, des membranes échangeuses d'ions ou bipolaires.

7. Procédé suivant l'une quelconque des revendications précédentes, **caractérisé en ce que** l'électrodialys est réalisée dans plusieurs étapes d'électrodialyse, disposées en série, dans lesquelles l'acide carboxylique di- ou polyoxydé est chaque fois, enrichi et amené à l'étape d'électrodialyse la plus proche.

8. Procédé suivant l'une quelconque des revendications précédentes, **caractérisé en ce que** l'on met en oeuvre pour les catalyseurs, des matériaux support poreux ou ceux avec une grande surface spécifique.

9. Procédé suivant l'une quelconque des revendications précédentes, **caractérisé en ce que** comme substance de départ, on met en oeuvre des saccharides non réduits, comme par exemple le saccharose, le tréhalose et/ou des saccharides réduits, comme par exemple le glucose, le palatinose, le fructose et/ou des alcools de sucre, comme par exemple le palatinitol, le sorbitol et/ou les dérivés monooxydés ou des mélanges ou les dérivés monooxydés des composés cités et on les dioxyde.

10. Procédé suivant l'une quelconque des revendications précédentes, **caractérisé en ce que** comme solvant pour les substances de départ, on met en oeuvre l'eau ou des mélanges d'eau et d'alcools secondaires, de préférence l'isopropanol.

11. Procédé suivant l'une quelconque des revendications précédentes, **caractérisé en ce que** l'oxydation et l'électrodialyse sont réalisées à un pH allant de 1 à 13.

12. Procédé suivant l'une quelconque des revendications précédentes, **caractérisé en ce que** la température pour l'oxydation se situe entre 0°C et 80°C.

13. Procédé suivant la revendication 12, **caractérisé en ce que** la température pour l'oxydation se situe entre 20°C et 60°C.

14. Procédé suivant l'une quelconque des revendications précédentes, **caractérisé en ce que** l'on met en oeuvre le réactif en une concentration allant de 3% à 20%.

15. Procédé suivant l'une quelconque des revendications précédentes, **caractérisé en ce que** pour ajuster le pH , on met en oeuvre Na₂CO₃, K₂CO₃ ou NaHCO₃ ou NaOH ou d'autres agents d'alcalinisation.

16. Dispositif pour réaliser l'un des procédés suivant l'une quelconque des revendications 1 à 15, **caractérisé en ce qu'**il présente, disposés en série, une étape d'absorption du gaz (10), une étape d'oxydation (30) et plusieurs étapes d'électrodialyse (40), ainsi que une conduite de recyclage (43) des étapes d'électrodialyse à l'étape d'absorption des gaz (10).
